# EUROPÄISCHE PATENTSCHRIFT

(11) **EP 3 388 854 B1**
(45) Veröffentlichungstag und Bekanntmachung des Hinweises auf die Patenterteilung: **16.09.2020**
(21) Anmeldenummer: 17166038.4
(22) Anmeldetag: 11.04.2017
(51) Int. Cl.: G01R 33/36, G01R 33/48, G01R 33/563

(54) **STANDARDISIERTE FUNKKOPPLUNG ZWISCHEN EINER MAGNETRESONANZANLAGE UND EINER EXTERNEN VORRICHTUNG**
STANDARDIZED WIRELESS COUPLING BETWEEN A MAGNETIC RESONANCE SYSTEM AND AN EXTERNAL DEVICE
COUPLAGE DE RADIOCOMMUNICATION NORMALISÉ ENTRE UNE INSTALLATION À RÉSONANCE MAGNÉTIQUE ET UN DISPOSITIF EXTERNE

(43) Veröffentlichungstag der Anmeldung: 17.10.2018
(73) Patentinhaber: Siemens Healthcare GmbH, 91052 Erlangen (DE)
(72) Erfinder: Franger, Dirk, 91052 Erlangen (DE); Hengerer, Arne, 91096 Möhrendorf (DE); Lauer, Lars, 91077 Neunkirchen (DE); Rothgang, Eva, 90571 Schwaig bei Nürnberg (DE); Schneider, Rainer, 91054 Erlangen (DE)

(56) Entgegenhaltungen:
- EP-A1- 3 196 715
- DE-A1-102015 200 220
- US-A1- 2003 058 502
- US-A1- 2005 107 681
- US-A1- 2014 275 970
- US-A1- 2017 065 823
- GUILHERME GUSTAVO RAMOS GOMES ET AL: "A Specification and Tool for the Configuration of REST Applications", ADVANCED INFORMATION NETWORKING AND APPLICATIONS WORKSHOPS, 2009. WAINA '09. INTERNATIONAL CONFERENCE ON, 26. Mai 2009 (2009-05-26), Seiten 500-505, XP055408995, Piscataway, NJ, USA DOI: 10.1109/WAINA.2009.185 ISBN: 978-1-4244-3999-7

## Beschreibung

Die vorliegende Erfindung betrifft eine Magnetresonanzanlage und ein System, welches eine Magnetresonanzanlage und eine externe Vorrichtung umfasst, wobei die Kommunikationsverbindung der externen Vorrichtung mit der Magnetresonanzanlage im Vordergrund steht.

Die nachveröffentlichte EP 3 196 715 A1 offenbart das Betreiben einer Magnetresonanzanlage durch eine externe Steuervorrichtung. Dabei umfasst die Magnetresonanzanlage eine drahtlose Schnittstelle, um über diese Schnittstelle eine Kommunikation mit der externen Steuervorrichtung zu betreiben. Die Schnittstelle ist insbesondere standardisiert und verwendet ein REST-basiertes HTTP-Protokoll.

Die US 2014/0275970 A1 beschreibt ein MRI-kompatibles Kommunikationssystem. Dabei verwaltet ein Schnittstellenmodul Kommunikationen zwischen Vorrichtungen. Dieses Schnittstellenmodul übersetzt Nachrichten zwischen verschiedenen drahtlosen Kommunikationsstandards. Das Kommunikationssystem überträgt Daten zwischen Sensoren und einer MRI-Steuerung.

Die DE 10 2015 200 220 A1 beschreibt ein Magnetresonanztomographiegerät mit einer Datenschnittstelle zum drahtlosen Empfangen von Steuerdaten von einer Anzeigevorrichtung. Dabei können Bilddaten von dem Magnetresonanztomographiegerät an die Anzeigevorrichtung drahtlos gesendet werden.

Die US 2005/0107681 A1 offenbart ein System zur drahtlosen Kommunikation von physiologischen Daten, welche den Zustand eines Patienten anzeigen, zu einem Scanner eines MR-Systems. Dabei können die drahtlosen Techniken auch eingesetzt werden, um eine Infusionseinrichtung zu steuern, welche mit dem Patienten verbunden ist.

In einem MR-Raum befinden sich neben dem MR-Scanner bzw. der Magnetresonanzanlage und Zubehörgerätschaften, wie Empfangsspulen oder Lagerungshilfen, Vorrichtungen, welche den MR-Untersuchungsprozess unterstützen oder ergänzen. Zu diesen externen Vorrichtungen gehören beispielsweise Kontrastmittel-Injektoren oder EKG-Geräte. Diese externen Vorrichtungen sind nicht Teil der Magnetresonanzanlage und werden meist auch separat vertrieben. Die externen Vorrichtungen können jedoch über einen externen Trigger-Eingang mit der Magnetresonanzanlage verbunden werden. Die Verbindung mit der Magnetresonanzanlage ermöglicht eine zeitliche Taktung der Aktivitäten (z.B. des Kontrastmittel-injektors) oder eine Aufzeichnung bestimmter Informationen (z.B. des EKG-Geräts). Ohne eine solche Kommunikationsverbindung zwischen beispielsweise dem Kontrastmittel-Injektor und der Magnetresonanzanlage müsste bei einer bezüglich des Kontrasts optimierten ("contrast enhanced") MR-Angiographie die Kontrastmittelgabe und der Start des MR-Protokolls manuell an zwei unabhängigen Konsolen erfolgen.

Nach dem Stand der Technik werden die externen Vorrichtungen über Kabel mit der Magnetresonanzanlage verbunden, sofern überhaupt eine physikalische Verbindung zwischen der externen Vorrichtung und der Magnetresonanzanlage erstellt wird. Aufgrund der räumlichen Enge im MR-Raum und der zum Teil zahlreichen unterschiedlichen Geräte bzw. Vorrichtungen ist die Anbindung über Kabel oft störend und teilweise sogar mit Risiken (Stolperfalle) verbunden.

Daher stellt sich die vorliegende Erfindung die Aufgabe, dieses Verbindungsproblem zwischen einer externen Vorrichtung und einer Magnetresonanzanlage zu lösen.

Erfindungsgemäß wird diese Aufgabe durch eine Magnetresonanzanlage nach Anspruch 1 oder 3 und durch ein System nach Anspruch 4 oder 6 gelöst.

Im Rahmen der vorliegenden Erfindung wird eine Magnetresonanzanlage bereitgestellt, welche ausgestaltet ist, um eine Untersuchung eines Untersuchungsobjekts durchzuführen. Dabei umfasst die Magnetresonanzanlage eine HF-Steuereinheit, eine Gradientensteuereinheit und eine Bildsequenzsteuerung, welche ausgestaltet sind, um MR-Daten eines Volumenabschnitts des Untersuchungsobjekts zu erfassen. Darüber hinaus ist eine Recheneinheit der Magnetresonanzanlage ausgestaltet, um MR-Bilder abhängig von den erfassten MR-Daten zu rekonstruieren. Schließlich ist eine standardisierte REST-basierte HTTP-Funkschnittstelle der Magnetresonanzanlage ausgestaltet, um eine standardisierte Funkverbindung zu einer externen Vorrichtung aufzubauen.

Unter der REST-basierten HTTP-Funkschnittstelle wird eine standardisierte Funkschnittstelle verstanden, welche ein REST ("Represential State Transfer")-basiertes HTTP-Protokoll verwendet, was der externen Vorrichtung vorteilhafterweise ermöglicht, eine beliebige Technik zur Verbindung mit der Magnetresonanzanlage einzusetzen, welche TCP/IP und HTTP unterstützt. Als Kommunikationsverbindung zu der externen Vorrichtung unterstützt die erfindungsgemäße Magnetresonanzanlage neben WLAN, WiFi, Bluetooth alle weiteren Arten von Internet-Verbindungen.

Anhand der REST-basierten HTTP-Funkschnittstelle existiert vorteilhafterweise für jede externe Vorrichtung eine bidirektionale und generische Schnittstelle zur Magnetresonanzanlage, welche es ermöglicht, beliebige externe Vorrichtungen einfach über eine drahtlose Netzwerkverbindung mit der Magnetresonanzanlage zu verbinden. Bei der REST-basierten HTTP-Funkschnittstelle wird eine Anforderung (Request) immer von der externen Vorrichtung (d.h. der Client-Seite) getriggert und eine Antwort (Response) auf diese Anforderung von der Magnetresonanzanlage zu der externen Vorrichtung zurückgeschickt.

In Kombination mit einem drahtlosen Netzwerk ermöglicht die REST-basierte HTTP-Funkschnittstelle somit auf einfache und generische Art und Weise eine kabellose Anbindung einer beliebigen externen Vorrichtung an die Magnetresonanzanlage. Die REST-basierte HTTP-Funkschnittstelle weist vorteilhafterweise auch eine gute Erweiterbarkeit auf, um für bestimmte Szenarien von externen Vorrichtungen maßgeschneiderte Dienste bereitzustellen. Daher stellt die vorliegende Erfindung eine einfache und schnelle Lösung für die drahtlose Anbindung von externen Vorrichtungen bereit, wobei durch die REST-basierte HTTP-Funkschnittstelle auf ein vorhandenes, generisches Schnittstellen-Framework zurückgegriffen wird, welches die Basis und den Rahmen für die Kommunikation zwischen einer externen Vorrichtung und der Magnetresonanzanlage zur Verfügung stellt. Damit stellt die vorliegende Erfindung aufgrund der erfindungsgemäßen Architektur der Magnetresonanzanlage eine einfache Erweiterungsmöglichkeit für zusätzliche Dienste (Services) bereit, welche für spezielle externe Vorrichtungen (Klienten) und deren Anforderungen maßgeschneidert werden können.

Es sei explizit darauf hingewiesen, dass die standardisierte Funkverbindung zwischen der Magnetresonanzanlage und der externen Vorrichtung auch mittels Protokollen bzw. Techniken, wie Protobuf ("Protocol Buffers", einem Datenformat zur Serialisierung mit einer Schnittstellen-Beschreibungssprache), ZeroMQ ((oft auch ØMQ, 0MQ oder ZMQ genannt), was eine sehr perfomante asynchrone Nachrichtensammlung ("messaging library") ist), RabbitMQ (implementiert ein Nachrichten-Warteschlangen-Protokoll, das als "Advanced Message Queuing Protocol" (AMQP) bekannt ist), usw. realisiert werden kann.

Eine erfindungsgemäße Magnetresonanzanlage ist in der Lage, in Abhängigkeit von dem Erfassen der MR-Daten eine Anweisung oder eine Information zu erstellen und diese Anweisung oder diese Information dann drahtlos über die REST-basierte HTTP-Schnittstelle an die externe Vorrichtung zu übertragen.

Diese Magnetresonanzanlage ermöglicht vorteilhafterweise, dass das Erfassen der MR-Daten mit einer Aktion der externen Vorrichtung (beispielsweise einem Kontrastmittel-Injektor) synchronisiert wird.

Die Anweisung und/oder die Information kann beispielsweise mittels so genannter WebSockets an die externe Vorrichtung gesendet werden.

Das Versenden von WebSockets basiert dabei insbesondere auf dem WebSocket-Protokoll. Das WebSocket-Protokoll ist ein auf TCP basierendes Netzwerkprotokoll, mit dem eine bidirektionale Verbindung zwischen der Magnetresonanzanlage (als WebSocket-Server) und der externen Vorrichtung möglich ist. Während bei einer reinen HTTP-Verbindung eine Information oder Anweisung von der Magnetresonanzanlage nur dann an die externe Vorrichtung gesendet werden kann, wenn die externe Vorrichtung diesbezüglich eine Anfrage an die Magnetresonanzanlage gestellt hat, reicht es beim WebSocket-Protokoll, wenn die externe Vorrichtung die Funkverbindung zur Magnetresonanzanlage öffnet. Die Magnetresonanzanlage kann dann diese offene Verbindung aktiv verwenden, um Informationen und/oder Anweisungen an die externe Vorrichtung zu senden, ohne dazu auf eine neue Verbindung der externen Vorrichtung warten zu müssen. Technisch betrachtet startet bei WebSocket, wie bei HTTP, die externe Vorrichtung eine Anfrage, mit dem Unterschied, dass nach der Übertragung der Daten zum Verbindungsaufbau die zugrundeliegende TCP-Verbindung bestehen bleibt und Übertragungen in beide Richtungen ermöglicht.

Darüber hinaus ist eine andere erfindungsgemäße Magnetresonanzanlage ausgebildet, um eine Information über die REST-basierte HTTP-Schnittstelle von der externen Vorrichtung zu erfassen und den Schritt der Erfassung der MR-Daten und/oder den Schritt einer Rekonstruktion von MR-Bildern abhängig von dieser Information durchzuführen.

Mit anderen Worten kann die externe Vorrichtung über die einmal erstellte Verbindung Informationen (z.B. EKG-Daten) an die erfindungsgemäße Magnetresonanzanlage übertragen. Die Magnetresonanzanlage kann dann die Erfassung der MR-Daten und/oder die Rekonstruktion der MR-Bilder an die derart übertragenen Informationen anpassen.

Im Rahmen der vorliegenden Erfindung wird auch ein System bereitgestellt, welches neben der vorab beschriebenen erfindungsgemäßen Magnetresonanzanlage auch eine oder mehrere externe Vorrichtungen umfasst.

Die Vorteile des erfindungsgemäßen Systems entsprechen im Wesentlichen den Vorteilen der erfindungsgemäßen Magnetresonanzanlage, welche vorab im Detail ausgeführt sind, so dass hier auf eine Wiederholung verzichtet wird.

Eine erfindungsgemäße externe Vorrichtung des erfindungsgemäßen Systems umfasst Steuermittel, Funkmittel und Sensormittel. Dabei sind die Funkmittel ausgestaltet, um eine standardisierte drahtlose Verbindung (z.B. WLAN, WiFi, Bluetooth) über die REST-basierte HTTP-Schnittstelle mit der Magnetresonanzanlage aufzubauen. Die Sensormittel sind ausgestaltet, um von dem zu untersuchenden Untersuchungsobjekt Informationen (z.B. EKG-Daten) zu erfassen. Die Steuermittel sind wiederum ausgestaltet, um diese Informationen mittels der Funkmittel über die REST-basierte HTTP-Schnittstelle an die Magnetresonanzanlage zu übertragen.

Wenn es sich bei der externen Vorrichtung beispielsweise um ein EKG-Gerät handelt, sind die Sensormittel ausgestaltet, um entsprechende EKG-Daten des Untersuchungsobjekts zu erfassen.

Eine andere erfindungsgemäße externe Vorrichtung des erfindungsgemäßen Systems umfasst Steuermittel, Funkmittel und Aktivierungsmittel. Während die Funkmittel ausgestaltet sind, um eine standardisierte drahtlose Verbindung über die REST-basierte HTTP-Schnittstelle mit der Magnetresonanzanlage aufzubauen, sind die Aktivierungsmittel ausgestaltet, um das Untersuchungsobjekt zu stimulieren oder dem Untersuchungsobjekt etwas zu verabreichen. Die Steuermittel sind gemäß dieser Ausführungsform ausgestaltet, um abhängig von einer Anweisung, welche über die Funkmittel und die REST-basierte HTTP-Schnittstelle von der Magnetresonanzanlage empfangen worden ist, die Aktivierungsmittel anzusteuern.

Für die Aktivierungsmittel existieren erfindungsgemäß folgende Varianten:
- Wenn es sich beispielsweise bei der externen Vorrichtung um einen Kontrastmittel-Injektor handelt, sind die Aktivierungsmittel ausgestaltet, um dem Untersuchungsobjekt ein Kontrastmittel zu verabreichen.
- Wenn die externe Vorrichtung zur MR-Elastographie eingesetzt werden soll, werden mit den Aktivierungsmitteln Druckwellen erzeugt, um diese Druckwellen auf einen bestimmten Bereich des Untersuchungsobjekts zu richten. Bei der MR-Elastographie wird der untersuchte Bereich, durch die von außen (d.h. von der externen Vorrichtung) einwirkenden Druckwellen zyklisch komprimiert und wieder entlastet, während synchron zu den Druckwellen bzw. der Aktivierung der Aktivierungsmittel die MR-Daten erfasst werden. Anhand der rekonstruierten MR-Bilder können dann Teilbereiche einer unterschiedlichen Elastizität aufgezeigt werden, so dass beispielsweise gutartige von bösartigen Tumoren unterschieden werden können.
- Die Aktivierungsmittel können zur Erzeugung von hochintensiven fokussierten Ultraschall (HIFU ("High Intensity Focused Ultrasound")) ausgestaltet sein, um diese Ultraschallwellen auf einen Bereich des Untersuchungsobjekts zu richten. Diese hochintensiven fokussierten Ultraschallwellen können zur Ultraschallablation oder zur Ultraschallchirurgie jeweils unter Kontrolle von mittels der Magnetresonanzanlage erzeugten MR-Bildern eingesetzt werden. Dabei ist häufig eine genaue Kontrolle der Erwärmung des mit den hochintensiven fokussierten Ultraschallwellen bestrahlten Bereichs erforderlich, was mit Temperatur-sensitiven Sequenzen mittels der Magnetresonanzanlage oder mittels MT-Thermometrie ausgeführt werden kann.
- Die Aktivierungsmittel können eine Ventilationsmaschine oder Beatmungsmaschine umfassen, um das Untersuchungsobjekt bei der Untersuchung mittels der Magnetresonanzanlage künstlich zu beatmen. Dadurch ist das erfindungsgemäße System auch in der Lage, eine Messung (ein Erfassen von MR-Daten) künstlich mit Atemanhalteperioden des Untersuchungsobjekts zu synchronisieren, indem die Aktivierungsmittel entsprechend angesteuert werden.

Bei allen vorab beschriebenen erfindungsgemäßen Varianten kann vorteilhafterweise durch die Funkverbindung zwischen der externen Vorrichtung und der Magnetresonanzanlage die Tätigkeit der Aktivierungsmittel mit der Erfassung der MR-Daten durch die Magnetresonanzanlage synchronisiert werden.

Gemäß einer weiteren erfindungsgemäßen Ausführungsform umfasst die Vorrichtung Steuermittel, Funkmittel und Eingabemittel. Wiederum sind die Funkmittel ausgestaltet, um eine standardisierte drahtlose Verbindung über die REST-basierte HTTP-Schnittstelle mit der Magnetresonanzanlage aufzubauen, während die Eingabemittel ausgestaltet sind, um eine Eingabe einer Bedienperson (z.B. eines die Magnetresonanzanlage bedienenden Mediziners) zu erfassen. Die Steuermittel sind bei dieser Ausführungsform ausgestaltet, um abhängig von der Eingabe eine Information mittels der Funkmittel der externen Vorrichtung über die REST-basierte HTTP-Schnittstelle an die Magnetresonanzanlage zu senden.

Bei dieser Ausführungsform kann der Mediziner der Magnetresonanzanlage vorteilhafterweise mitteilen, dass ein bestimmter Zustand vorliegt, indem der Mediziner zu dem entsprechenden Zeitpunkt die Eingabemittel (z.B. ein einfaches Fußpedal) betätigt. Eine entsprechende externe Vorrichtung ist auch als State-Publisher bekannt.

Das erfindungsgemäße System (insbesondere die Magnetresonanzanlage) ist in der Lage, die Eingabe oder die von der Eingabe abhängige Information, welche von der einen der externen Vorrichtungen empfangen wird, an eine oder mehrere andere der externen Vorrichtungen weiterzuleiten. Damit wird quasi ein State-Publishing-Mechanismus realisiert. Dadurch bekommen beispielsweise eine erste und eine zweite der externen Vorrichtungen mit, dass die Eingabemittel (z.B. ein Knopf) bei der dritten der externen Vorrichtungen betätigt wurden.

Gemäß einer weiteren erfindungsgemäßen Ausführungsform umfasst die eine oder die andere erfindungsgemäße Vorrichtung Steuermittel, Funkmittel und Ausgabemittel. Wiederum sind die Funkmittel ausgestaltet, um eine standardisierte drahtlose Verbindung über die REST-basierte HTTP-Schnittstelle mit der Magnetresonanzanlage aufzubauen, während die Ausgabemittel ausgestaltet sind, um eine Information auszugeben. Dabei sind die Steuermittel ausgestaltet, um eine weitere Information, welche über die Funkmittel der externen Vorrichtung über die REST-basierte HTTP-Schnittstelle von der Magnetresonanzanlage empfangen worden ist, über die Ausgabemittel als die Information auszugeben.

Bei dieser Ausführungsform kann eine beliebige Information von der Magnetresonanzanlage mittels der Ausgabemittel der externen Vorrichtung ausgegeben werden, um beispielsweise den die Magnetresonanzanlage bedienenden Mediziner über einen bestimmten Zustand der Magnetresonanzanlage (beispielsweise das Ende der Datenerfassung) zu informieren.

Es sei explizit darauf hingewiesen, dass die eine oder die andere externe Vorrichtung keine Steuerung der Magnetresonanzanlage darstellt. Daher überträgt die externe Vorrichtung auch keine Anweisung an die Magnetresonanzanlage, welche dann von der Magnetresonanzanlage auszuführen wäre.

Insbesondere kann es sich bei jeder der vorab beschriebenen externen Vorrichtungen um eine MR-kompatible externe Vorrichtung handeln. Dabei wird unter einer MR-kompatiblen Vorrichtung eine Vorrichtung verstanden, welche ohne Einschränkung ihrer Funktionalität im Umfeld einer Magnetresonanzanlage eingesetzt werden kann, insbesondere auch dann, wenn die Magnetresonanzanlage MR-Daten erfasst. Eine MR-kompatible externe Vorrichtung stört also weder die Magnetresonanzanlage beim Erfassen von MR-Daten noch wird die MR-kompatible externe Vorrichtung von der Magnetresonanzanlage durch das Schalten von Gradienten und Einstrahlen von HF-Pulsen (beim Erfassen von MR-Daten) gestört.

Die eine oder die andere externe Vorrichtung kann auch zur Ausführung eines Informationsdienstes, eines Patientendatendienstes, eines Interaktionsdienstes und/oder eines Autorisierungsdienstes ausgestaltet sein. Diese Dienste können über die REST-basierte HTTP-Schnittstelle der Magnetresonanzanlage bereitgestellt werden.

Mit dem Informationsdienst kann die externe Vorrichtung bestimmte Informationen der Magnetresonanzanlage erfassen. Diese Informationen umfassen beispielsweise
- eine Liste von Sequenzen, welche von der Magnetresonanzanlage ausgeführt werden können,
- einen aktuellen Zustand (z.B. laufend, offen, angehalten) einer bestimmten oder ausgewählten Sequenz, und
- eine verbleibende Messzeit einer aktuell laufenden Sequenz.

Der Patientendatendienst stellt der externen Vorrichtung bestimmte Informationen, wie den Namen, das Alter, die Körperlänge oder das Gewicht des Patienten, bereit.

Über den Interaktionsdienst wird eine Meldung oder ein Hinweis, welcher von der Magnetresonanzanlage ausgegeben wird, an die externe Vorrichtung weitergeleitet. Über diese Meldung oder diesen Hinweis kann die externe Vorrichtung über eine Aktion der Magnetresonanzanlage informiert werden, welche beispielsweise einen Dialogeingriff eines Benutzers erfordert.

Der Autorisierungsdienst erfasst mit Autorisierungsdaten, welche selbst in der Regel eine Lizenz umfassen, Informationen darüber, welche Funktionen der externen Vorrichtung von einer bestimmten Bedienperson ausgeführt werden dürfen. Durch den Autorisierungsdienst kann sehr genau vorgegeben werden, welche Funktion der jeweiligen externen Vorrichtung von dem jeweiligen Benutzer für die Magnetresonanzanlage freigegeben werden darf, so dass diese Funktion dann von der Magnetresonanzanlage ausgeführt werden darf oder Informationen an die Magnetresonanzanlage übertragen darf.

Die Erfindung wird nachfolgend unter Bezugnahme auf die beiliegenden Zeichnungen näher erläutert.

In Fig. 1 ist schematisch ein erfindungsgemäßes System mit einer erfindungsgemäßen Magnetresonanzanlage und vier externen Vorrichtungen dargestellt.

In Figuren 2 und 3 ist jeweils ein Flussplan dargestellt, um den Betrieb eines erfindungsgemäßen Systems zu erläutern.

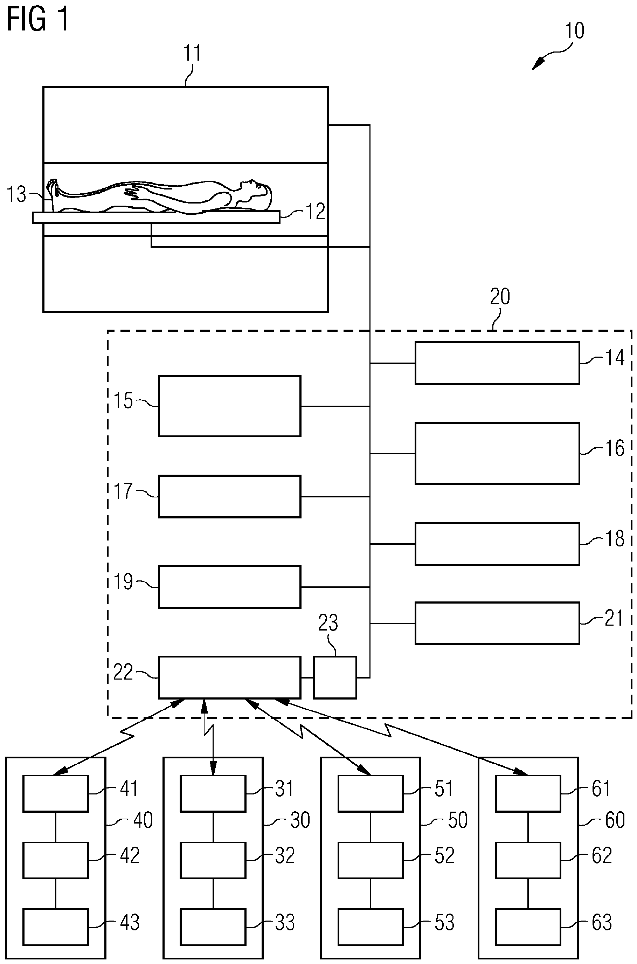

In Fig. 1 ist ein erfindungsgemäßes System dargestellt, welches eine erfindungsgemäße Magnetresonanzanlage 10 und vier externe Vorrichtungen 30-60 umfasst. Die Magnetresonanzanlage 10 weist einen Magneten 11 zur Erzeugung eines Polarisationsfelds B0 auf, wobei eine auf einer Liege 12 angeordnete Untersuchungsperson 13 in den Magneten 11 gefahren wird, um dort ortskodierte Magnetresonanzsignale aus der Untersuchungsperson 13 aufzunehmen.

Die Magnetresonanzanlage 10 weist weiterhin eine Steuereinheit 20 auf, die zur Steuerung der Magnetresonanzanlage 10 verwendet werden kann. Die Steuerung 20 weist eine Gradientensteuereinheit 15 zur Steuerung und Schaltung der notwendigen Magnetfeldgradienten auf. Eine HF-Steuereinheit 14 ist für die Steuerung und Generierung der HF-Pulse zur Auslenkung der Magnetisierung vorgesehen. Eine Bildsequenzsteuerung 16 steuert die Abfolge der Magnetfeldgradienten und HF-Pulse und damit indirekt die Gradientensteuereinheit 15 und die HF-Steuereinheit 14. Über eine Eingabeeinheit 17 kann eine Bedienperson die Magnetresonanzanlage 10 steuern, und auf einer Anzeigeeinheit 18 können MR-Bilder und sonstige zur Steuerung notwendigen Informationen angezeigt werden. Eine Recheneinheit 19 mit mindestens einer Prozessoreinheit (nicht gezeigt) ist vorgesehen zur Steuerung der verschiedenen Einheiten in der Steuereinheit 20. Weiterhin ist eine Speichereinheit 21 vorgesehen, in der beispielsweise Programmmodule bzw. Programme abgespeichert sein können, die, wenn sie von der Recheneinheit 19 bzw. ihrer Prozessoreinheit ausgeführt werden, den Ablauf der Magnetresonanzanlage 10 steuern können.

Schließlich umfasst die Magnetresonanzanlage 10 Funkmittel 22 und eine standardisierte REST-basierte HTTP-Funkschnittstelle 23, um eine standardisierte drahtlose Verbindung zu jeder der externen Vorrichtungen 30-60 aufzubauen. Die externe Vorrichtung 30 umfasst Funkmittel 31, Steuermittel 32 und Sensormittel 33. Mit den Sensormitteln 33 kann die externe Vorrichtung 30 Informationen über das Untersuchungsobjekt 13 erfassen, so dass es sich bei der externen Vorrichtung 30 beispielsweise um ein EKG-Gerät handeln kann.

Die externe Vorrichtung 40 umfasst neben Funkmittel 41 und Steuermitteln 42 Aktivierungsmittel 43, mit welchen die externe Vorrichtung 40 das Untersuchungsobjekt 13 stimulieren kann oder dem Untersuchungsobjekt 13 etwas verabreichen kann. Daher kann es sich bei der externen Vorrichtung 40 um einen Kontrastmittel-Injektor, um ein Gerät zur MR-Elastographie, um ein Gerät zum Erzeugen von hochintensivem fokussiertem Ultraschall oder um eine Ventilationsmaschine handeln.

Auch die externe Vorrichtung 50 umfasst Steuermittel 52 und Funkmittel 51. Zusätzlich umfasst die externe Vorrichtung 50 Eingabemittel 53, um eine Eingabe einer Bedienperson zu erfassen, welche dann als eine entsprechende Information mittels der Steuermittel 52 und der Funkmittel 51 an die Magnetresonanzanlage 10 übertragen wird.

Schließlich umfasst die externe Vorrichtung 60 Funkmittel 61, Steuermittel 62 und Ausgabemittel 63. Die externe Vorrichtung 60 ist ausgestaltet, um mit Hilfe der Steuermittel 62 eine Information, welche die externe Vorrichtung 60 mittels ihrer Funkmittel 61 von der Magnetresonanzanlage 10 empfangen hat, über die Ausgabemittel auszugeben.

Durch die Funkmittel 22 und die REST-basierte HTTP-Funkschnittstelle 23 der Magnetresonanzanlage 10 kann vorteilhafterweise sehr einfach eine kabellose Anbindung der externen Vorrichtungen 30-60 an die Magnetresonanzanlage 10 erfolgen. Dadurch kann die Magnetresonanzanlage 10 vorteilhafterweise synchron mit jeder der externen Vorrichtungen 30-60 zusammenarbeiten. Dadurch ist bei dem in Fig. 1 dargestellten erfindungsgemäßen System ein automatisch abgestimmter Betrieb zwischen der Magnetresonanzanlage 10 und jeder der externen Vorrichtungen 30-60 möglich.

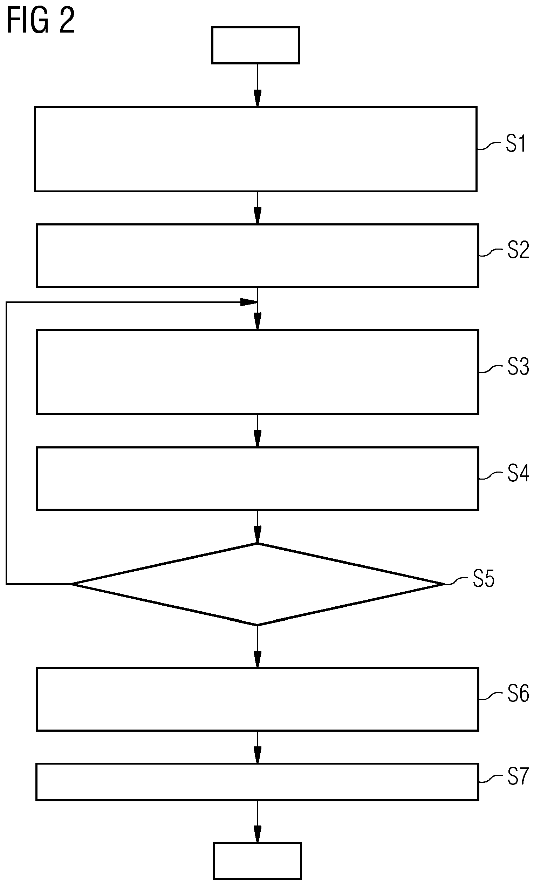

In Fig. 2 ist der Flussplan eines Vorgehens dargestellt, mit welchem der Betrieb des in Fig. 1 dargestellten erfindungsgemäßen Systems erläutert werden soll.

In Schritt S1 wird ein externer Kontrastmittel-Injektor 40 drahtlos über die REST-basierte HTTP-Funkschnittstelle 23 mit der Magnetresonanzanlage 10 verbunden. Im Schritt S2 beginnt die Magnetresonanzanlage 10 MR-Daten eines Patienten 13 zu erfassen. Abgestimmt mit der Erfassung der MR-Daten steuert im Schritt S3 die Magnetresonanzanlage 10 mittels WebSockets automatisch den Kontrastmittel-Injektor an, um ein Kontrastmittel in den Körper des Patienten 13 zu injizieren. Auch nach dem Beginn der Injektion des Kontrastmittels werden im Schritt S4 weiter MR-Daten des Patienten mit Hilfe der Magnetresonanzanlage 10 erfasst. Solange im Schritt S5 nicht entschieden wird, die Injektion des Kontrastmittels zu beenden, wird das Kontrastmittel im Schritt S3 weiter injiziert und MR-Daten im Schritt S4 weiter erfasst.

Nach Beendigung der Kontrastmittelgabe werden im Schritt S6 weiter MR-Daten des Patienten 13 mittels der Magnetresonanzanlage 10 erfasst. Abschließend werden MR-Bilder aus den MR-Daten im Schritt S7 rekonstruiert.

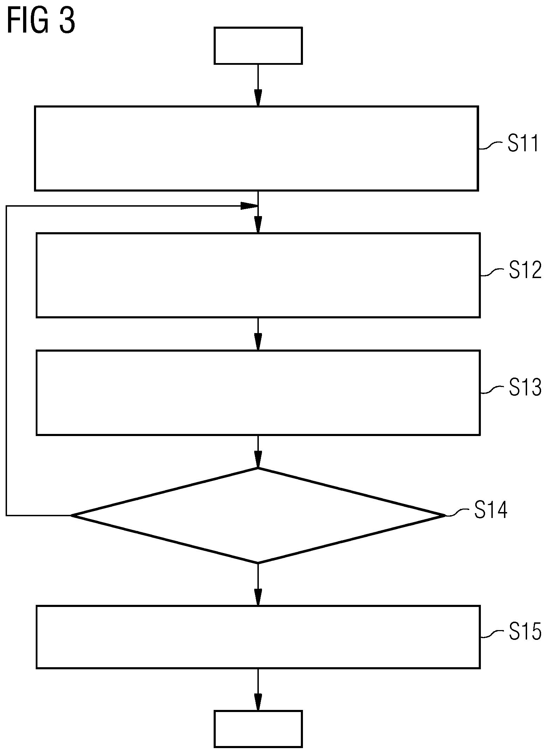

In Fig. 3 ist der Flussplan eines weiteren Vorgehens dargestellt, mit welchem eine weitere Variante des Betriebs des in Fig. 1 dargestellten erfindungsgemäßen Systems erläutert werden soll.

Bei dieser Variante wird ein externes EKG-Gerät 30 im Schritt S11 drahtlos über die REST-basierte HTTP-Funkschnittstelle 23 mit der Magnetresonanzanlage 10 verbunden. Mit diesem externen EKG-Gerät 30 werden im Schritt S12 EKG-Daten (d.h. der Herzschlag) eines Patienten 13 erfasst und über die im Schritt S11 aufgebaute drahtlose Kommunikationsverbindung an die Magnetresonanzanlage 10 übertragen. Die Magnetresonanzanlage 10 erfasst diese EKG-Daten im Schritt S13 und erfasst abhängig von diesen EKG-Daten MR-Daten des Patienten 13. Die Schritte S12 und S13 werden so lange wiederholt durchgeführt, bis im Schritt S14 entschieden wird, dass Erfassen von MR-Daten zu beenden. Schließlich werden im Schritt S15 aus den MR-Daten MR-Bilder rekonstruiert, wobei dabei die erfassten EKG-Daten berücksichtigt werden.

## Patentansprüche

1. Magnetresonanzanlage (10), die ausgebildet ist, um eine Untersuchung eines Untersuchungsobjekts (13) durchzuführen, wobei die Magnetresonanzanlage (10) umfasst:
eine HF-Steuereinheit (14), eine Gradientensteuereinheit (15) und eine Bildsequenzsteuerung (16), die ausgebildet sind, um MR-Daten eines Volumenabschnitts des Untersuchungsobjekts (13) zu erfassen, und
eine Recheneinheit (19), die ausgebildet ist, um MR-Bilder ausgehend von den erfassten MR-Daten zu rekonstruieren,
wobei die Magnetresonanzanlage (10) ausgebildet ist, um zum einen abhängig von dem Erfassen der MR-Daten eine Anweisung oder Information zu erstellen, insbesondere das Erfassen der MR-Daten mit einer Aktion einer Vorrichtung (40; 60) zu synchronisieren, und um zum anderen die Anweisung oder Information über eine Schnittstelle (23) an die Vorrichtung (40; 60) zu senden, welche dazu eingerichtet ist, einen MR-Untersuchungsprozess zu unterstützen oder zu ergänzen,
**dadurch gekennzeichnet,**
**dass** die Schnittstelle (23) eine standardisierte REST-basierte HTTP-Funkschnittstelle (23) der Magnetresonanzanlage (10) ist, die ausgebildet ist, um eine standardisierte drahtlose Verbindung zu der Vorrichtung (40; 60) aufzubauen.

2. Magnetresonanzanlage nach Anspruch 1,
dass die Magnetresonanzanlage (10) ausgebildet ist, um mittels WebSockets die Anweisung und/oder Information über die Schnittstelle (23) an die Vorrichtung (40; 60) zu senden.

3. Magnetresonanzanlage (10), die ausgebildet ist, um eine Untersuchung eines Untersuchungsobjekts (13) durchzuführen, wobei die Magnetresonanzanlage (10) umfasst:
eine HF-Steuereinheit (14), eine Gradientensteuereinheit (15) und eine Bildsequenzsteuerung (16), die ausgebildet sind, um MR-Daten eines Volumenabschnitts des Untersuchungsobjekts (13) zu erfassen, und
eine Recheneinheit (19), die ausgebildet ist, um MR-Bilder ausgehend von den erfassten MR-Daten zu rekonstruieren,
wobei die Magnetresonanzanlage (10) ausgebildet ist, um eine Information über eine Schnittstelle (23) von einer Vorrichtung (30), welche dazu eingerichtet ist, einen MR-Untersuchungsprozess zu unterstützen oder zu ergänzen, zu erfassen und das Erfassen der MR-Daten und/oder die Rekonstruktion der MR-Bilder abhängig von der Information durchzuführen,
wobei die Information von Sensormitteln (33) der Vorrichtung (30) von dem Untersuchungsobjekt (13) erfasst ist,
**dadurch gekennzeichnet,**
**dass** die Schnittstelle (23) eine standardisierte REST-basierte HTTP-Funkschnittstelle (23) der Magnetresonanzanlage (10) ist, die ausgebildet ist, um eine standardisierte drahtlose Verbindung zu der Vorrichtung (30) aufzubauen.

4. System mit einer Magnetresonanzanlage (10) gemäss Anspruch 3, mit einer Vorrichtung (30),
wobei die Vorrichtung (30), welche einen MR-Untersuchungsprozess unterstützt oder ergänzt, Steuermittel (32), Funkmittel (31) und Sensormittel (33) umfasst,
wobei die Sensormittel (33) ausgestaltet sind, um eine Information von dem Untersuchungsobjekt (13) zu erfassen,
wobei die Steuermittel (32) ausgestaltet sind, um die Information mittels der Funkmittel (31) über die Schnittstelle (23) an die Magnetresonanzanlage (10) zu senden, wobei die Funkmittel (31) ausgestaltet sind, um eine standardisierte drahtlose Verbindung über die Schnittstelle (23) mit der Magnetresonanzanlage (10) aufzubauen.

5. System nach Anspruch 4,
**dadurch gekennzeichnet,**
**dass** die Sensormittel (33) ausgestaltet sind, um eine EKG-Information von dem Untersuchungsobjekt (13) zu erfassen.

6. System mit einer Magnetresonanzanlage (10) gemäss Anspruch 1, mit einer Vorrichtung (40; 60),
wobei die Vorrichtung (40; 60), welche dazu eingerichtet ist, einen MR-Untersuchungsprozess zu unterstützen oder zu ergänzen, Steuermittel (42), Funkmittel (41) und Aktivierungsmittel (43) umfasst, wobei die Aktivierungsmittel (43) ausgestaltet sind, um das Untersuchungsobjekt (13) zu stimulieren oder dem Untersuchungsobjekt (13) etwas zu verabreichen,
wobei die Steuermittel (42) ausgestaltet sind, um abhängig von einer Anweisung, welche mittels der Funkmittel (41) über die Schnittstelle (23) von der Magnetresonanzanlage (10) empfangen wird, die Aktivierungsmittel (43) anzusteuern, wobei die Funkmittel (41) ausgestaltet sind, um eine standardisierte drahtlose Verbindung mit der Magnetresonanzanlage (10) aufzubauen.

7. System nach Anspruch 6,
**dadurch gekennzeichnet,**
**dass** die Aktivierungsmittel (43) ausgestaltet sind, um dem Untersuchungsobjekt (13) ein Kontrastmittel zu verabreichen.

8. System nach Anspruch 6,
**dadurch gekennzeichnet,**
**dass** die Aktivierungsmittel (43) zur MR-Elastographie ausgestaltet sind, indem die Aktivierungsmittel (43) Druckwellen erzeugen, um diese auf einen Bereich des Untersuchungsobjekts (13) zu richten.

9. System nach Anspruch 6,
**dadurch gekennzeichnet,**
**dass** die Aktivierungsmittel (43) zur Erzeugung von hoch intensivem fokussiertem Ultraschall ausgestaltet sind, um diesen Ultraschall auf einen Bereich des Untersuchungsobjekts (13) zu richten.

10. System nach Anspruch 7,
**dadurch gekennzeichnet,**
**dass** die Aktivierungsmittel (43) eine Ventilationsmaschine umfassen, um das Untersuchungsobjekt (13) künstlich zu beatmen.

11. System nach Anspruch 4 oder 6,
**dadurch gekennzeichnet,**
**dass** die Vorrichtung (50) Eingabemittel (53) umfasst, dass die Eingabemittel (53) ausgestaltet sind, um eine Eingabe einer Bedienperson zu erfassen, dass die Steuermittel (52) ausgestaltet sind, um abhängig von der Eingabe eine Information mittels der Funkmittel (51) über die Schnittstelle (23) an die Magnetresonanzanlage (10) zu senden.

12. System nach Anspruch 4 oder 6,
**dadurch gekennzeichnet,**
**dass** die Vorrichtung (60) Ausgabemittel (63) umfasst, dass die Ausgabemittel (63) ausgestaltet sind, um eine Information auszugeben,
**dass** die Steuermittel (62) ausgestaltet sind, um eine weitere Information, welche mittels der Funkmittel (61) über die Schnittstelle (23) von der Magnetresonanzanlage (10) empfangen wird, über die Ausgabemittel (63) als die Information auszugeben.

13. System nach einem der Ansprüche 4-12,
**dadurch gekennzeichnet,**
**dass** die Vorrichtung (30; 40; 60) MR-kompatibel ist.

14. System nach einem der Ansprüche 4-13,
**dadurch gekennzeichnet,**
**dass** die Vorrichtung (30; 40; 60) zur Ausführung eines Informationsdienstes, eines Patientendatendienstes, eines Interaktionsdienstes und/oder eines Autorisierungsdienstes ausgestaltet ist,
wobei der Informationsdienst zum Ausgeben einer Liste von Sequenzen, welche von der Magnetresonanzanlage (10) ausführbar sind, zum Ausgeben eines aktuellen Zustands einer bestimmten Sequenz und/oder zum Erfassen einer verbleibenden Messzeit einer aktuell auf der Magnetresonanzanlage (10) laufenden Sequenz ausgestaltet ist,
wobei der Patientendatendienst Informationen über einen Patienten (13) bereitstellt,
wobei der Interaktionsdienst zur Ausgabe eines Hinweises oder einer Meldung der Magnetresonanzanlage (10) ausgestaltet ist, und
wobei der Autorisierungsdienst anhand von Autorisierungsdaten bestimmt, welche Funktion der Vorrichtung (30; 40; 60) von einer bestimmten Bedienperson ausgeführt werden darf.

## Claims

1. Magnetic resonance system (10), which is designed to carry out an examination of an examination object (13),
wherein the magnetic resonance system (10) comprises:
an RF control unit (14), a gradient control unit (15) and an image sequence controller (16), which are designed to acquire MR data of a volume portion of the examination object (13), and
an arithmetic unit (19), which is designed to reconstruct MR images starting from the acquired MR data,
wherein the magnetic resonance system (10) is designed on the one hand to create an instruction or information as a function of the acquisition of the MR data, in particular to synchronise the acquisition of the MR data with an action of a device (40; 60), and on the other hand to send the instruction or information to the device (40; 60) via an interface (23), which device is set up to aid or complement an MR examination process,
**characterised in that**
the interface (23) is a standardised REST-based HTTP radio interface (23) of the magnetic resonance system (10), which is designed to establish a standardised wireless connection to the device (40; 60).

2. Magnetic resonance system according to claim 1,
that the magnetic resonance system (10) is designed to send the instruction and/or information to the device (40; 60) via the interface (23) by way of WebSockets.

3. Magnetic resonance system (10), which is designed to carry out an examination of an examination object (13), wherein the magnetic resonance system (10) comprises:
an RF control unit (14), a gradient control unit (15) and an image sequence controller (16), which are designed to acquire MR data of a volume portion of the examination object (13), and
an arithmetic unit (19), which is designed to reconstruct MR images starting from the acquired MR data,
wherein the magnetic resonance system (10) is designed to acquire an item of information via an interface (23) from a device (30), which is set up to aid or complement an MR examination process, and to carry out the acquisition of the MR data and/or the reconstruction of the MR images as a function of the information,
wherein the information is acquired by sensor means (33) of the device (30) from the examination object (13),
**characterised in that**
the interface (23) is a standardised REST-based HTTP radio interface (23) of the magnetic resonance system (10), which is designed to establish a standardised wireless connection to the device (30).

4. System having a magnetic resonance system (10) according to claim 3, and with a device (30),
wherein the device (30), which aids or complements an MR examination process, comprises control means (32), radio means (31) and sensor means (33),
wherein the sensor means (33) are configured to acquire an item of information from the examination object (13),
wherein the control means (32) are configured to send the information to the magnetic resonance system (10) via the interface (23) by way of the radio means (31),
wherein the radio means (31) are configured to establish a standardised wireless connection to the magnetic resonance system (10) via the interface (23).

5. System according to claim 4,
**characterised in that**
the sensor means (33) are configured to acquire ECG information from the examination object (13).

6. System having a magnetic resonance system (10) according to claim 1, with a device (40; 60),
wherein the device (40; 60), which is set up to aid or complement an MR examination process, comprises control means (42), radio means (41) and activation means (43),
wherein the activation means (43) are configured to stimulate the examination object (13) or to administer something to the examination object (13),
wherein the control means (42) are configured to control the activation means (43) as a function of an instruction, which is received from the magnetic resonance system (10) via the interface (23) by way of the radio means (41),
wherein the radio means (41) are configured to establish a standardised wireless connection to the magnetic resonance system (10).

7. System according to claim 6,
**characterised in that**
the activation means (43) are configured to administer a contrast medium to the examination object (13).

8. System according to claim 6,
**characterised in that**
the activation means (43) are configured for MR elastography in that the activation means (43) generate shock waves in order to direct these onto a region of the examination object (13) .

9. System according to claim 6,
**characterised in that**
the activation means (43) are configured for generating highly intensive, focused ultrasound in order to direct this ultrasound onto a region of the examination object (13).

10. System according to claim 7,
**characterised in that**
the activation means (43) comprise a ventilation machine in order to artificially respirate the examination object (13).

11. System according to claim 4 or 6,
**characterised in that**
the device (50) comprises input means (53),
the input means (53) are configured to detect an input by an operator,
the control means (52) are configured to send an item of information to the magnetic resonance system (10) via the interface (23) by way of the radio means (51) as a function of the input.

12. System according to claim 4 or 6,
**characterised in that**
the device (60) comprises output means (63),
the output means (63) are configured to output an item of information,
as the information, the control means (62) are configured to output a further item of information via the output means (63), which information is received from the magnetic resonance system (10) via the interface (23) by way of the radio means (61).

13. System according to one of claims 4-12,
**characterised in that**
the device (30; 40; 50; 60) is MR compatible.

14. System according to one of claims 4-13,
**characterised in that**
the device (30; 40; 60) is configured for carrying out an information service, a patient data service, an interaction service and/or an authorisation service,
wherein the information service is configured for outputting a list of sequences, which can be performed by the magnetic resonance system (10), for outputting a current state of a particular sequence and/or for acquiring a remaining scan time of a sequence currently running on the magnetic resonance system (10),
wherein the patient data service provides information about a patient (13),
wherein the interaction service is configured for outputting an indication or a message from the magnetic resonance system (10), and
wherein, using authorisation data, the authorisation service determines which function of the device (30; 40; 60) can be carried out by a particular operator.

## Revendications

1. Installation (10) de résonance magnétique, constituée pour effectuer un examen d'un objet (13) à examiner, l'installation (10) de résonance magnétique comprenant :
une unité (14) de commande HF, une unité (15) de commande de gradient et une commande (16) de séquence d'image, qui sont constituées pour saisir des données RM d'une partie du volume de l'objet (13) à examiner, et
une unité (19) informatique, constituée pour reconstruire des images RM à partir des données RM saisies,
l'installation (10) de résonance magnétique étant constituée d'une part pour produire une instruction ou une information qui dépend de la saisie des données RM, notamment pour synchroniser la saisie des données RM avec une action d'un dispositif (40 ; 60), et d'autre part pour envoyer l'instruction ou l'information par l'intermédiaire d'une interface (23) au dispositif (40 ; 60), qui est conçu pour venir à l'appui d'une opération d'examen RM ou pour la compléter,
**caractérisée**
**en ce que** l'interface (23) est une interface (23) radio HTTP normalisée basée sur REST de l'installation (10) de résonance magnétique, qui est constituée pour établir une liaison sans fil normalisée avec le dispositif (40 ; 60).

2. Installation de résonance magnétique suivant la revendication 1,
dans laquelle l'installation (10) de résonance magnétique est constituée pour envoyer, au moyen de Web Socket l'instruction et/ou l'information au dispositif (40 ; 60) par l'intermédiaire de l'interface (23).

3. Installation (10) de résonance magnétique constituée pour effectuer un examen d'un objet (13) à examine, l'installation (10) de résonance magnétique comprenant :
une unité (14) de commande HF, une unité (15) de commande de gradient et une commande (16) de séquence d'image, qui sont constituées pour saisir des données RM d'une partie du volume de l'objet (13) à examiner, et
une unité (19) informatique, constituée pour reconstruire des images RM à partir des données RM saisies,
l'installation (10) de résonance magnétique étant constituée pour saisir une information par l'intermédiaire d'une interface (23) d'un dispositif (30) conçu pour venir à l'appui d'une opération d'examen RM ou pour la compléter et pour effectuer la saisie des données RM et/ou la reconstruction des images RM en fonction de l'information,
dans laquelle l'information est saisie de l'objet (13) à examiner par des moyens (33) de capteur du dispositif (30),
**caractérisée en ce que**
**en ce que** l'interface (23) est une interface (23) radio HTTP normalisée basée sur REST de l'installation (10) de résonance magnétique, qui est constituée pour établir une liaison sans fil normalisée avec le dispositif (30).

4. Système comprenant une installation (10) de résonance magnétique suivant la revendication 3,
comprenant un dispositif (30),
le dispositif (30), qui vient à l'appui d'une opération d'examen RM ou la complète, comprenant des moyens (32) de commande, des moyens (31) radio et des moyens (33) de capteur,
les moyens (33) de capteur étant conformés pour saisir de l'information de l'objet (13) à examiner,
les moyens (32) de commande étant conformés pour envoyer l'information à l'installation (10) de résonance magnétique à l'aide des moyens (31) radio par l'intermédiaire de l'interface (23),
dans lequel
les moyens (31) radio sont conformés pour établir une liaison sans fil normalisée par l'intermédiaire de l'interface (23) avec l'installation (10) de résonance magnétique.

5. Système suivant la revendication 4,
**caractérisé**
**en ce que** les moyens (33) de capteur sont conformés pour saisir une information ECG de l'objet (13) à examiner.

6. Système comprenant une installation (10) de résonance magnétique suivant la revendication 1,
comprenant un dispositif (40 ; 60),
dans lequel le dispositif (40 ; 60), qui est conçu pour venir à l'appui d'une opération d'examen RM ou pour la compléter, comprend des moyens (42) de commande, des moyens (41) radio et des moyens (43) d'activation,
les moyens (43) d'activation étant conformés pour stimuler l'objet (13) à examiner ou pour administrer quelque chose à l'objet (13) à examiner,
les moyens (42) de commande étant conformés pour, en fonction d'une instruction, qui est reçue à l'aide des moyens (41) radio par l'installation (10) de résonance magnétique par l'intermédiaire de l'interface (23), commander les moyens (43) d'activation,
les moyens (41) radio étant conformés pour établir une liaison sans fil normalisée avec l'installation (10) de résonance magnétique.

7. Système suivant la revendication 6,
**caractérisé**
**en ce que** les moyens (43) d'activation sont constitués pour administrer un agent de contraste à l'objet (13) à examiner.

8. Système suivant la revendication 6,
**caractérisé**
**en ce que** les moyens (43) d'activation sont constitués pour l'élastographie RM par le fait que les moyens (43) d'activation produisent des ondes de pression, afin de les diriger sur une partie de l'objet (13) à examiner.

9. Système suivant la revendication 6,
**caractérisé**
**en ce que** les moyens (43) d'activation sont confirmés pour la production d'un ultrason focalisé très intense, conformés, afin de diriger cet ultrason sur une partie de l'objet (13) à examiner.

10. Système suivant la revendication 7,
**caractérisé**
**en ce que** les moyens (43) d'activation comprennent une machine de ventilation pour faire respirer artificiellement l'objet (13) à examiner.

11. Système suivant la revendication 4 ou 6,
**caractérisé**
**en ce que** le dispositif (50) comprend des moyens (53) d'entrée,
**en ce que** les moyens (53) d'entrée sont conformés pour saisir une entrée d'un opérateur,
**en ce que** les moyens (52) de commande sont conformés pour, en fonction de l'entrée, envoyer une information à l'aide des moyens (51) radio à l'installation (10) de résonance magnétique par l'intermédiaire de l'interface (23).

12. Système suivant la revendication 4 ou 6,
**caractérisé**
**en ce que** le dispositif (60) comprend des moyens (63) d'émission,
**en ce que** les moyens (63) d'émission sont conformés pour émettre une information,
**en ce que** les moyens (62) de commande sont conformés pour émettre, par les moyens (63) de sortie comme information, une autre information, qui est reçue à l'aide des moyens (61) radio par l'installation (10) de résonance magnétique par l'intermédiaire de l'interface (23).

13. Système suivant l'une des revendications 4 à 12,
**caractérisé**
**en ce que** le système (30 ; 40 ; 60) est compatible à la RM.

14. Système suivant l'une des revendications 4 à 13,
**caractérisé**
**en ce que** le dispositif (30 ; 40 ; 60) est conformé pour la réalisation d'un service d'information, d'un service de données de patient, d'un service d'interaction et/ou d'un service d'autorisation,
dans lequel le service d'information est conformé pour l'émission d'une liste de séquences, qui peuvent être réalisées par l'installation (10) de résonance magnétique pour l'émission d'un état présent d'une séquence déterminée et/ou pour la saisie d'un temps de mesure restant d'une séquence se déroulant présentement sur l'installation (10) de résonance magnétique,
dans lequel le service de données du patient met à disposition des informations sur un patient (13),
dans lequel le service d'interaction est conformé pour l'émission d'une indication ou d'un message de l'installation (10) de résonance magnétique, et
dans lequel le service d'autorisation détermine, à l'aide de données d'autorisation, la fonction du dispositif (30 ; 40 ; 60), qui doit être exécutée par un opérateur déterminé.
